# EUROPEAN PATENT SPECIFICATION

(11) **EP 2 064 556 B1**
(45) Date of publication and mention of the grant of the patent: **10.05.2017**
(21) Application number: 07827651.6
(22) Date of filing: 30.08.2007
(51) Int. Cl.: G01N 33/68, G01N 33/86, G01N 33/94, C12Q 1/68, A61K 31/616

(54) **METHOD TO IDENTIFY AND TREAT SUBJECTS RESISTANT TO ACETYL SALICYLIC ACID**
VERFAHREN ZUR IDENTIFIZIERUNG UND BEHANDLUNG VON GEGEN ACETYLSALICYLSÄURE RESISTENTEN PATIENTEN
MÉTHODE D'IDENTIFICATION ET DE TRAITEMENT DE SUJETS RÉSISTANTS À L'ACIDE ACÉTYLSALICYLIQUE

(30) Priority: 31.08.2006 IT RM20060406
(43) Date of publication of application: 03.06.2009
(73) Proprietor: Pulcinelli, Fabio Maria, 00136 Roma (RM) (IT); Frati, Luigi, 00136 Roma (RM) (IT)
(72) Inventor: PULCINELLI, Fabio Maria, 00185 Roma RM (IT); FRATI, Luigi, 00185 Roma RM (IT); MATTIELLO, Teresa, 00185 Roma RM (IT)
(74) Representative: Trupiano, Federica
(86) International application number: PCT/IT2007/000597
(87) International publication number: WO 2008/026234

(56) References cited:
- WO-A-2005/044244
- JEDLITSCHKY G ET AL: "The nucleotide transporter MRP4 (ABCC4) is highly expressed in human platelets and present in dense granules, indicating a role in mediator storage" BLOOD, W.B.SAUNDERS COMPANY, ORLANDO, FL, vol. 104, no. 12, 5 August 2004 (2004-08-05), pages 3603-3610, XP002319468 ISSN: 0006-4971
- LENZ T L ET AL: "Aggrenox: a fixed-dose combination of aspirin and dipyridamole." THE ANNALS OF PHARMACOTHERAPY NOV 2000, vol. 34, no. 11, November 2000 (2000-11), pages 1283-1290, XP009101611 ISSN: 1060-0280
- SZTRIHA L K ET AL: "Aspirin resistance in stroke: 2004" JOURNAL OF NEUROLOGICAL SCIENCES, ELSEVIER SCIENTIFIC PUBLISHING CO, AMSTERDAM, NL, vol. 229-230, 15 March 2005 (2005-03-15), pages 163-169, XP004781207 ISSN: 0022-510X
- UCHIYAMA SHINICHIRO ET AL: "NEW MODALITIES AND ASPECTS OF ANTIPLATELET THERAPY FOR STROKE PREVENTION" CEREBROVASCULAR DISEASES, KARGER, BASEL, CH, vol. 21, no. SUPPLEMENT 1, 1 January 2006 (2006-01-01), pages 7-16, XP008070361 ISSN: 1015-9770
- HANKEY ET AL: "Aspirin resistance" LANCET THE, LANCET LIMITED. LONDON, GB, vol. 367, no. 9510, 18 February 2006 (2006-02-18), pages 606-617, XP005297138 ISSN: 0140-6736
- SEREBRUANY V ET AL: "DIPYRIDAMOLE DECREASES PROTEASE-ACTIVATED RECEPTOR AND ANNEXIN-V BINDING ON PLATELETS OF POSTSTROKE PATIENTS WITH ASPIRIN NONRESPONSIVENESS" CEREBROVASCULAR DISEASES, KARGER, BASEL, CH, vol. 21, no. 1-2, 1 January 2006 (2006-01-01), pages 98-105, XP008070363 ISSN: 1015-9770
- MASON ET AL: "Aspirin Resistance and Atherothrombotic Disease" JOURNAL OF THE AMERICAN COLLEGE OF CARDIOLOGY, ELSEVIER, NEW YORK, NY, US, vol. 46, no. 6, 20 September 2005 (2005-09-20), pages 986-993, XP005407553 ISSN: 0735-1097
- A MÉNDEZ, R HERKLOTZ, AR HUBER: "Aspirinresistenz?ein komplexes, aber ernstzunehmendes Phänomen" CURRICULUM, [Online] no. 6, 2006, pages 898-904, XP002484515 Schweiz Med Forum Retrieved from the Internet: URL:http://www.medicalforum.ch/pdf/pdf_d/2 006/2006-40/2006-40-128.PDF> [retrieved on 2008-06-17]
- REID G ET AL: "The human multidrug resistance protein MRP4 functions as a prostaglandin efflux transporter and is inhibited by nonsteroidal antiinflammatory drugs" PROCEEDINGS OF THE NATIONAL ACADEMY OF SCIENCES OF USA, NATIONAL ACADEMY OF SCIENCE, WASHINGTON, DC, vol. 100, no. 16, 5 August 2003 (2003-08-05), pages 9244-9249, XP002973737 ISSN: 0027-8424
- MATTIELLO T., GUERRIERO R., LOTTI L., GAUDIO C., FRATI L., PUCINELLI F.M.: "EVIDENCE OF ASPIRIN EXCRETION FROM PLATELET BY MRP4 TRANSPORT RESPONSIBLE FOR REDUCED DRUG ACTIVITY IN PATIENTS AT 5 DAYS CABS", JOURNAL OF THROMBOSIS AND HAEMOSTASIS 2007, vol. 5, no. S2, O-T-070, 1 August 2007 (2007-08-01),
- RICHARD FITZGERALD ET AL: 'Aspirin resistance: Effect of clinical, biochemical and genetic factors' PHARMACOLOGY & THERAPEUTICS vol. 130, no. 2, 01 May 2011, pages 213 - 225, XP055020074 DOI: 10.1016/j.pharmthera.2011.01.011 ISSN: 0163-7258
- TERESA MATTIELLO ET AL: 'Aspirin Extrusion From Human Platelets Through Multidrug Resistance Protein-4-Mediated Transport' JOURNAL OF THE AMERICAN COLLEGE OF CARDIOLOGY vol. 58, no. 7, 01 August 2011, pages 752 - 761, XP055019876 DOI: 10.1016/j.jacc.2011.03.049 ISSN: 0735-1097
- JOHN W. EIKELBOOM ET AL: 'Overexpression of the Multidrug Resistance Protein-4 Transporter in Patients Undergoing Coronary Artery Bypass Graft Surgery' JOURNAL OF THE AMERICAN COLLEGE OF CARDIOLOGY vol. 58, no. 7, 01 August 2011, pages 762 - 764, XP055020077 DOI: 10.1016/j.jacc.2011.04.025 ISSN: 0735-1097
- WHITE ET AL: "TIME WAITS FOR NO MAN: DECIDING WHEN TO FILE A PATENT APPLICATION IN EUROPE", NATURE BIOTECHNOLOGY, NATURE PUBLISHING GROUP, NEW YORK, NY, US, vol. 25, no. 6, 1 June 2007 (2007-06-01), pages 639-641, XP009085067, ISSN: 1087-0156, DOI: 10.1038/NBT0607-639

## Description

### Field of the invention

The present invention relates to the use of acetylsalicylic acid in combination with MRP4 channel inhibitors for the treatment of patients resistant to said acetylslicylic acid. This invention is also related to a method of *in vitro* analysis and its corresponding kit, to indentify such patients

### Background art

For about thirty years now particular laboratory and clinical evidence has been established for a condition which immediately revealed itself to be a marked interindividual difference in the response to therapy with acetylsalicylic acid (ASA) (hereinafter the term ASA will be used to denote acetylsalicylic acid, its derivatives and corresponding physiologically acceptable salts) (Mason et al. J. Am. Coll. Cardiol. 2005, 46, 986-93 and Hankey and Eikelboom The Lancet 2006, 367, 606-17), accompanied by a progressive reduction in its efficacy in long-term therapy (Pulcinelli et al. J. Am. Coll. Cardiol. 2004, 43, 979-84). This evidence has been defined as "ASA resistance", and currently represents a problem, the actual clinical extent of which has yet to be demonstrated and which continues to occupy the time and interest of many researchers.

ASA resistance is identified as the inability of this molecule to fully inhibit the production of thromboxane-A2 (TxA2) or TxA2-dependent platelet activation (biochemical or laboratory resistance). This resistance may consequently be responsible for the failure of therapy to prevent cardiovascular events in patients on chronic treatment with ASA (clinical resistance) (Hankey and Eikelboom, ref. cited).

The approaches adopted to reduce this phenomenon are two and are based on two types of intervention: increasing the daily doses of aspirin or combining aspirin with another drug with an anti-platelet action, but both approaches have failed to prove effective; in fact, in the former case, it has been demonstrated that increasing the aspirin dose (above 150 mg/day) increases the risk of cardiovascular complications as compared to patients taking 75-150 mg/day (ATC Br. Med. J. 2002, 324, 71-86); in the latter case, it has been established that while this therapeutic approach is useful in secondary prevention, i.e., after a cardiovascular event, it may be risky in primary prevention, i.e., in patients with a high risk of atherothrombotic complications who have previously not suffered cardiovascular events, in that it may increase the risk of haemorrhage.

Aspirin®, the active ingredient of which is acetylsalicylic acid (ASA), is the drug most sold world-wide; as an analgesic it belongs to the class of the NSAIDs (Non-Steroidal Anti-Inflammatory Drugs) and is the anti-aggregant drug *par excellence* in the treatment of subjects who have suffered acute coronary syndromes or who are at high risk of cerebro-cardiovascular events or in the treatment of obliterating arteropathies of the peripheral vessels. Among the numerous studies indicating the efficacy of ASA in reducing atherothrombotic diseases, we may cite a recent meta-analysis conducted on 287 randomised trials by the "Antiplatelet Trialists' Collaboration" (ATC Br. Med. J. 2002, 324, 71-86): this study has demonstrated that a daily intake of ASA at low doses (75-150 mg/day) reduces the risk of cardiovascular complications (myocardial infarction, cerebral infarction and vascular death) by 25% in patients who have previously had coronary disease. ASA is present in more than thirty pharmaceutical specialties, is prepared in various forms and can be administered via various different routes.

The importance of this drug in reducing atherothrombotic complications stems from the fact that such pathologies present the ischaemic event as a result of platelet activation.

Platelets are cell fragments that derive from the fragmentation of megakaryocytes. The morphology of these cells appears to be complex, presenting at their ends a glycocalix consisting of numerous membrane glycoproteins. More internally, microtubules and microfilaments make up the cytoskeleton, which has numerous granules embedded within it, belonging to three distinct groups: α-granules, dense granules and λ-granules. The α-granules, which are present in abundance, contain important proteins for coagulation and for the regeneration of damaged tissue. The dense granules contain chemical mediators such as ADP, ATP, serotonin and adrenalin, which mediate the amplification of the platelet response. The λ-granules are identifiable as lysosomes. The scanty presence of RNA gives rise to a reduced representation of the rough endoplasmatic reticulum, whereas the smooth endoplasmatic reticulum, referred to as the Dense Tubular System, is present in abundance and is responsible for the mobilisation of intracellular calcium.

The platelets play the physiological role of being activated following vascular damage and forming the first stage in arresting vascular rupture. In particular, following endothelial damage, the platelets rapidly adhere to the newly exposed collagen fibres, are activated and release chemical mediators: ADP, released by the dense granules, and thromboxane-A2 (TxA2), produced by the activation of COX-1, which activate other platelets at the site of the lesion, forming the platelet aggregate that blocks the bleeding. This cascade of events is entirely akin to that encountered in the pathological event of such activation, which is the formation of the platelet thrombus; in fact, in a vessel whose lumen is reduced due to the presence of an atheroma in the subendotheliun, events which are still unknown bring about the fissuration of the plaque, with exposure of the collagen fibres and activation of platelets, which by forming the aggregate within the vessel impede the passage of red blood cells downstream of the lesion. This interruption of the blood flow will therefore be the cause of the ischaemic pathology, which may be transient or may give rise to necrosis of the tissue, leading to events such as angina or myocardial infarction, if the lesion occurs in the coronary district, TIA (transient ischaemic attack) or stroke, if the lesion occurs in arteries supplying blood to the brain.

Therefore, secretion of the granules and the production of thromboxane are two very important platelet events for regulating the formation of the platelet thrombus. And it is for this reason that the two main drugs so far used to reduce cardiovascular events are aimed at reducing platelet activation by ADP (thienopyridine) and the production of thromboxane (ASA).

The efficacy of ASA in reducing platelet functionality is based on the inhibition of the cyclo-oxygenase activity of prostaglandin H synthase, an enzyme present in platelets and commonly defined as COX-1.

The mechanism of action of ASA consists in the acetylation of the amino acid Ser 529 of COX-1, which impedes the access of the substrate, arachidonic acid, to the catalytic site. It is therefore a matter of irreversible enzyme inhibition.

ASA is rapidly absorbed in the stomach and small bowel after oral administration: the plasma concentration peak is registered after 30-40 minutes. The half-life is very very short, amounting to just 15-20 minutes. Platelet inhibition, on the other hand, is manifested one hour after administration. Bioavailability is 40-50%, but this parameter does not influence the effect on platelets, since COX-1 is acetylated in the presystemic circulation.

Considering that platelets have no nucleus, they are incapable of synthesising new enzyme: since ASA inhibits thromboxane-dependent function irreversibly, and considering that the mean life of platelets is 10 days, only 10% of platelets presents fully active COX-1 24 hours after taking the drug, and for this reason a daily intake of ASA reduces the production of thromboxane almost entirely.

In any event, the residual activity of platelet COX-1 following treatment with ASA is still unknown. Presumably it varies from one subject to another.

Clinical and laboratory evidence has shown that not all patients taking ASA benefit from the therapy; for this reason the expression "ASA resistance" has been coined. A recent study of ours (Pulcinelli et al. J. Thromb. Haemost. 2005, 3, 2784-9) has demonstrated that underlying this phenomenon in some patients on chronic ASA treatment there may be the inability of this drug to effectively inhibit platelet COX-1, allowing the synthesis of low concentrations of TxA2, albeit sufficient to induce platelet activation.

By way of confirmation of this hypothesis we may cite a HOPE substudy in which Eikelboom et al. (Circulation 2002, 105, 1650-5) observed a positive correlation between the concentration of 11-deihyro-thromboxane-B₂ in the urine of patients treated with ASA and the related risk of infarction. Furthermore, Zimmerman et al. (Circulation 2003, 108, 542-7) detected the presence of a transient resistance to ASA due to impaired inhibition of COX-1, in pazients undergoing coronary bypass, with risks of cardiovascular complications for these patients. In none of these studies, however, has a treatment protocol capable of reducing this phenomenon been hypothesised.

Diabetic patients also appear to be less sensitive to the action of ASA, related to a reduced action on COX-1; in fact, one clinical trial (PPP study Sacco et al. Diabetes Care 2003, 26, 3264-72) has shown that diabetic patients on chronic ASA treatment have more cardiovascular complications than non-diabetic patients and we ourselves have recently demonstrated that the production of platelet thromboxane in diabetics treated with ASA is approximately 5 times greater than that in non-diabetic patients (Pulcinelli et al. J.A.C.C. 47(4) supplement A, 364A-365A).

Multidrug resistance proteins (MRPs) are a family of seven membrane glycoproteins, MRPs 1 to 7, which mediate the unidirectional transport of organic anions in the cells. In platelets, it has been demonstrated that type-4 MRPs are present and have the function of transporting adenylic nucleotides into the dense granules; the MRP4s, in fact, belong to the membrane protein family called ABC (ATP-binding cassette)-transporters (Jedlitschky et al. Blood 2004, 104, 3603-3610) and are responsible for the unidirectional transport of organic anions from the cytosol either towards the outside or towards the interior of the dense granules.

The fact that patients affected by certain diseases, diabetics or patients submitted to surgical operations, e.g., coronary bypass, show resistance to ASA prompted us to postulate that the cause of the reduced ability of this drug to inhibit platelet function may depend on the reduced accumulation of the drug in the cell due to its being transported outside the cytosol via the MRP4s (which therefore act as "passage channels", from which the name "MRP4 channels) and that this phenomenon may consequently lead to a reduction in its pharmacological activity.

The mechanism whereby the MRP4 channel inhibitors function is known (WO 2005/044244) and dipyridamole is mentioned among the inhibitors.

Dipyridamole is normally used in patients with coronary insufficiency accompanied or not by angina crises; coronaropathies; senile heart and the prophylaxis of myocardial infarction; cardiopathies, as a coadjuvant of digitalis therapy; diseases at cardiac, cerebral and renal level, due to increased platelet aggregability, since it is endowed with pronounced anti-platelet-aggregation and therefore antithrombotic activity, attributable to its platelet phosphodiesterase inhibiting activity which gives rise to an increase in AMPc. There is no suggestion in the literature that dipyridamole can increase the efficacy of ASA in inhibiting COX-1.

Combinations of dipyridamole and ASA are known and used in therapies related to cardiovascular diseases. In particular, US Patent 4694024 describes a system consisting in the release in succession first of dipyridamole and then of acetylsalicylic acid. US Patent 6015577, on the other hand, describes the simultaneous combined use of acetylsalicylic acid and dipyridamole, using lower doses of acetylsalicylic acid compared to US 4694024 and establishes the ideal ratio of dipyridamole to ASA for reducing the formation of coaguli in mice and therefore postulating a beneficial effect in the treatment of cardiovascular diseases.

Both patents refer to combinations of ASA and dipyridamole or similar drugs and are aimed at determining what the best dose of the two pharmacological ingredients is, what the best ratio is between the two ingredients and what are the best salts for bringing about a delay in the absorption of both. However, neither of them mentions or suggests the treatment of patients who prove resistant to ASA, nor that there exists a resistance caused by a reduced availability of ASA related, or which may be related, to the action of MRP4s. All that is said is that the ASA-dipyridamole combination is useful because both inhibit platelets, without any mention of MRP4s.
The pharmacology, pharmacokinetics, efficacy and safety of a fixed dose combination of aspirin and extended-release dipyridamole (Aggrenox'ID) for the secondary prevention of stroke has already been reviewed by Lenz T.L. and Hilleman D.E (Annals Pharmacotherapy (2000) 34: 1283-1290), while the phenomenon of aspirin resistance with reference to its effects in stroke has been described by Sztriha L.K. et al. (J. Neurol. Sci. (2005) 229-230: 163-169) and by Uchiyama S. et al (Cerebrovasc. Dis. (2006) 21 (suppl. 1): 7-16). These works concentrate on the prevention of stroke and atherothrombotic vascular disease and on the antiplatelet activity of dipyridamole with no mention of the ability of dipyridamole to inhibit MRP4 transport protein and of a possible role of the latter in aspirin resistance.

Also Serebruany et al. (Cerebrovasc. Dis. (2006) 21: 98-105) describe the antiplatelet activity of the combined administration of dipyridamole and aspirin in post-stroke patients with aspirin resistance, with no reference to MRP4 transport of ASA in platelets and to dipyridamole inhibition of MRP4-mediated transport. Aspirin resistance is also discussed in Mendez A. et al. (Curriculum (Online) (2006) 6: 898-904, Schweiz Med. Forum).
Reid et al. (PNAS (2003) 100: 9244-9249) describe the role of MRP4 protein in prostaglandin release from cells and hypothesize that nonsteroidal anti-inflammatory drugs might exert their action by inhibiting this prostaglandin release.

### Summary of the invention

It has now been found by the inventors that ASA is transported via the MRP4 channels; and it has also been found experimentally that a different expression of MRP4s is present in patients submitted to bypass, diabetics and in patients on chronic ASA treatment as compared to healthy volunteers.

Thus the inventors hypothesise that the cell, in the presence of massive doses of ASA, such as those administered to patients undergoing bypass surgery, or in the presence of disease conditions such as diabetes or in patients at high risk of thrombotic events on chronic treatment with aspirin, seeks to reduce the concentration of organic anionic substances in the cytosol, exporting part of them from the cytosol to the outside and another part from the cytosol into the granules. ASA, which is at physiological pH, presents as an organic anion, taking on a negative net charge on its surface, would thus constitute a molecular target for MRP4s.

This phenomenon has been proved by the inventors who verified the involvement of MRP4 channels in altering the intracellular path of ASA by measuring cytosolic concentrations of ASA and its metabolite salicylic acid (AS) or concentrations of these inside the platelet granules, in a platelet combination or in a preparation of platelet granules, as performed according to the method reported by Fukami MH et al. (J. Cell. Biol. 1978, 77, 389-99), both treated with ASA (50 □M for 10 minutes at 37°C or 4°C) or by means of the use of 14Q. labelled ASA, employing specific MRP4 inhibitors, such as dipyridamole and MK-571 ((E)-3-[[[3-[2-(7-chloro-2-quinolinyl)ethenyl]phenyl]-[[3-dimethylamino)-3-oxo-propyl]-thio]methyl] thio]-propanoic acid), and selective investigation procedures such as HPLC, for measuring ASA and SA according to the method described by Gaspari and Locatelli (Ther Drug Monit. 1987, 9, 243-7) and a beta-counter for measuring the radioactive asprin.

The inventors then found that inhibiting MRP4-mediated transport is advantageous in treatment with ASA, in that it reduces its extrusion from the cytosol, thereby increasing its ability to inhibit COX-1. This second hypothesis was verified by assaying thromboxane-A2 (TxA2), a marker of COX-1 activation, released by aspirinated and nonaspirinated platelets, activated with thrombin.

One object of the present invention is the use of acetylsalicylic acid, its derivatives and corresponding salts, and of MRP4 inhibitors, in combination or in succession, for the treatment of ASA resistance, as defined above. Preferred among the MRP4 inhibitors are dipyridamole and MK-571.

Another object of the invention relates to the daily doses and the administration protocol which may advantageously consist in daily doses of approximately 40 mg each of ASA and approximately 75 mg of dipyridamole.

Another object of the invention relates to the relative amounts of ASA and dipyridamole, which may range, respectively, from 75 to 150 mg/day in the case of ASA and from 75 to 500 mg/day, preferably 75 to 200 mg/day, in the case of dipyridamole.

Another object of the invention relates to the method and the use of a kit for the detection of patients resistant to ASA, including the resistances originating from genetic polymorphisms.

The invention is defined by the claims.

### Brief description of the figures

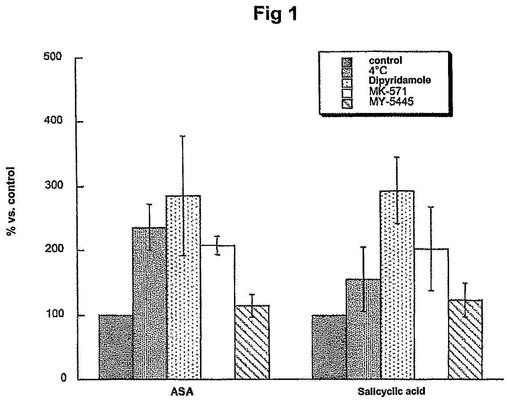

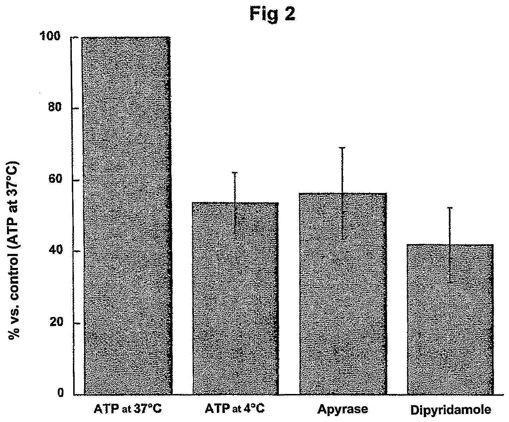

Figures 1 and 2 present the results obtained which are representative of the elimination of ASA from platelets by means of the MRP4s, achieved by measuring the concentrations of ASA and its metabolite salicylic acid in the cytosol (Fig. 1) and in the platelet granules (Fig. 2) which had been incubated with aspirin after different treatments, such as inhibitors of the transport mediated by the MRP4s (dipyridamole and MK-571), or systems that reduced the active transport of such channels (incubation at 4°C or with ATP scavengers) and recording the percentage obtained as compared to platelets or granules treated with ASA alone.
Figure 3 illustrates the release of 14C-labelled ASA after activation of platelets radiolabelled with thrombin.
Figure 4 illustrates the MRP4 expression in a platelet protein lysate of healthy volunteers as compared to patients undergoing bypass surgery at 5 days after the operation.
Figure 5 illustrates experiments conducted using the immunogold technique, which reveals the location of the channel by means of electron microscopy; the resistant patients present an increased cell concentration of the channel (panel B), in comparison with healthy volunteers (panel A).

### Detailed description of the invention

According to the present invention a combination of acetylsalicylic acid, its derivatives and physiologically acceptable salts, and MRP4 channel inhibitors, formulable in co-administration, is used for the treatment of ASA-resistant patients, as defined above.

The medicament according to the present invention can be obtained by mixing the active ingredients or their corresponding derivatives and pharmaceutically acceptable salts with excipients suitable for compositions for oral or parenteral administration, or for intramuscular, venous, per os or nasal administration. All the excipients, diluents, vehicles and/or adjuvants necessary for the preparation are ones with which the expert in the field will be familiar.

More specifically, what is meant by the term "composition" is a combination of active ingredients that is capable of producing a pharmacological effect as indicated above. The "composition" includes - in addition to the active ingredients - excipients, diluents, vehicles and/or adjuvants which alone are not capable of producing a pharmacological effect on the subject receiving the composition. These excipients, diluents, vehicles and adjuvants are well known to the experts in the field and can be selected from among the group of substances such as water, saline solution, glycerol, ethanol, emulsifying agents, buffer substances, preservatives and the like.

Examples of pharmaceutical compositions which may comprise the combination of active ingredients according to the invention are those in solid, semisolid or liquid form, in the form of lozenges, capsules, pills, tablets, pastilles, granulates, syrups, ampoules, drops, solutions, suspensions, emulsions, in unitary dosage forms or in separate doses, ready-to-use syrups or ex tempore units, creams and gels. Other examples of formulations are those for parenteral use, injectable forms for intramuscular, subcutaneous, transdermal or intravenous administration.

The compositions can be administered per os or parenterally, rectally or cutaneously.

Co-administration means a pack containing unitary or separate administration forms of the active ingredients, accompanied by the instructions for simultaneous coordinated administration or administration in succession of the active ingredients according to the dosage prescribed by the physician.

Administration doses, posologies and protocols are decided by the physician on the basis of his or her own experience, the severity of the disease and the patient's general condition and age.

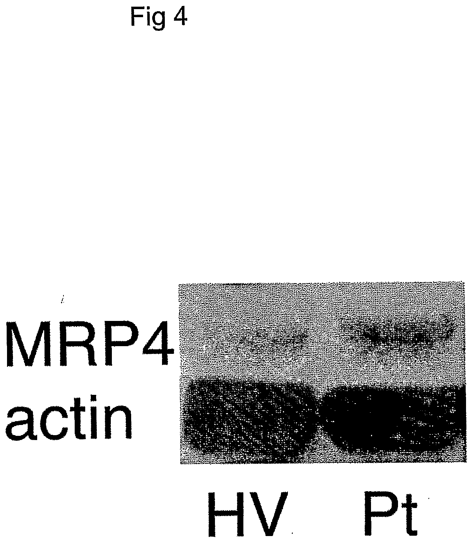

Patients manifesting ASA resistance according to the present invention present a higher platelet expression of MRP4 compared to healthy controls (Fig. 4).

A diagnostic method for the *in-vitro* detection of ASA-resistant subjects is claimed in claim 1. A way to carry out the method comprises the following steps:
a. Take a sample of biological fluid from the patient, generally purified venous peripheral blood, from which a platelet protein lysate is obtained which, after purification, i.e. through electrophoresis, is put in contact with anti-MRP4 mono- or polyclonal antibodies (available on the market) in conditions and for a time period sufficient for the formation of a complex between antigen (MRP4) and antibody;
b. Quantify the presence of the characteristic band of the MRP4s, the identification can be done by means of Western Blot by making a comparison with a control standard made up of the MRP4 protein.

The Western Blot technique is well known to the expert in the field, w ho, on the basis of the present description and his or her knowledge, can easily identify the optimal conditions for implementing the method.

Experts in the field are familiar with the definitions of "polyclonal antibody" and "monoclonal antibody".

Another mode of identification is based on the study of platelet messenger RNA responsible for the synthesis of this protein (MRP4), which is greater in resistant subjects than in healthy subjects. In this case the determination can be done using the Southern Blot technique or Real-Time PCR (RT-PCR), both of which are well known to the expert in the field, who will easily identify the necessary restriction enzymes and the subsequent conditions for electrophoresis.

A detection kit as disclosed may advantageously contain, in addition to the material for performing the assay (assay test tubes, titration plate wells, reagents, etc.) and the explanations relating to the method of use, anti-MRP4 antibodies for an evaluation assay by means of Western Blot; or specific restriction enzymes for the MRP4s (particularly profitable the TaqMan Gene Expression Assay kit) to be quantitatively analysed to determine platelet mRNA by Southern Blot or Re al-Time PCR.

The kit can suitably further comprise:
- a system for the purification of platelets from the other granular blood components (Platelet GelSep of BioCytex for purifications carried out with centrifugation methods on gel); and
- Reagents capable of quantifying the interferences due to non platelet components in the cellular preparation to be used in diagnosis such as monoclonal antibodies or anti CD45 restriction enzymes which reveal the presence of leucocytes.

As for the evaluation of the results that can be obtained with the method of the restriction enzymes, we can hypothesize that values higher than 20 UA (Arbitrare Units), preferably higher than 25 UA, more preferably higher than 30 UA, and even more preferably higher than 35 UA of threshold cycles of MRP4/GAPDH are values that identify resistant patients. Considering the pre-analytic and analytic variabilities of the lab data obtained with the kit for mRNA, it is recommended to create your own normal values, that can be obtained after analyzing at least 15 haelthy volunteers. As for the kit with western blot, we suggest the acquisition of data from resistant patients together with those of healthy volunteers. So, it is better add to the kit a standard preparation of MRP4 with a known value and the patient will be considered positive with a proteic densitometry higher than 10%, preferably 20%, better 35% and much better 50% compared to the standard.

Through the method of analysis of the invention and the corresponding kit, it is possibile to identify and monitor the subjects whose platelets show high levels of MRP4, this is of fundamental importance in reducing the level of therapeutic failures linked to aspirin chronic treatment. The kit advantageously allows to identify both patients ASA resistant and subjects who show such resistance because of genetic causes.

On the basis of the hypotheses advanced, the inventors have experimentally demonstrated that ASA can be conveyed via MRP4 channels and in patients with increased platelet expression of this protein, a reduced capability of ASA in reducing the platelet functionality is present and as a consequence inhibition of MRP4-mediated transport increases the therapeutic capacity induced by ASA.

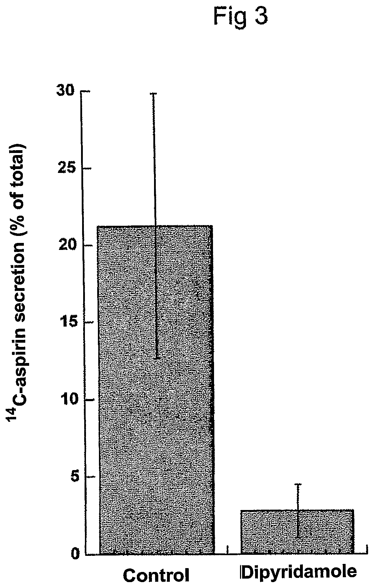

The fact that ASA can be transported into the dense granules or extruded from the cell via the MRP4s is demonstrated by the following experimental results:
1) inhibition of MRP4-mediated transport increases the ability of platelets to retain ASA in the cytosol; in fact, if the platelets are pretreated with dipyridamole or MK-571 10 minutes before incubation of the platelets with ASA 50 microM, the cytosolic concentrations of acetylsalicylic acid (ASA) or of its metabolite salicylic acid are 285% and 293% greater, respectively, in the platelets pretreated with dipyridamole 100 microM, whereas they are 208% and 202%, respectively, in the platelets treated with MK-571 as compared to platelets treated with the solvent alone both of dipyridamole and of MK-571 (DMSO, dimethylsulphoxide) (see Figure 1). Dipyridamole, in addition to inhibiting the transport mediated by these channels, is also an inhibitor of platelet activity, in that it increases the platelet concentrations of AMPc, inhibiting phosphodiesterase isoform V (PDE typeV). The inventors have demonstrated that the action of dipyridamole in inhibiting MRP4-mediated transport does not occur via inhibition of PDE type V, in that another inhibitor of this enzyme, 1-(3-chlorophenylamino)-4-phenylphthalazine (MY-5445), molecular formula C₂OH₁₄ClN₃, does not increase the cytosolic concentrations of ASA or salicylate (110% and 120%, respectively) (see Figure 1);
2) treatment of the platelets with ASA at a temperature below body temperature (4°C) increases the cytosolic concentrations of ASA and salicylate in the platelets by 240% and 160%, respectively: this demonstrates that the MRP4-mediated transport of ASA is efficient at body temperature (approximately 37°C) (see Figure 1);
3) ASA is internalised within the platelet granules via the MRP4s; this effect was demonstrated by assaying ASA in the granules after these organelles had been treated with ASA; that the transport can also occur via the MRP4s is demonstrated by the fact that it is necessary that the transport should occur at 37°C and in the presence of ATP in the external medium, in that MRP4-mediated transport is an active transport. In fact, the maximum concentration reached was obtained if ATP was added to the external medium and the incubation with ASA was done at 37°C, whereas it was substantially reduced if the incubation of ASA was done at 4°C (53.6%), in the presence of apyrase (56.1%), an enzyme which degrades ATP, or if the platelets were pretreated with dipyridamole (41.7%) 10 minutes prior to the addition of ASA (see Figure 2);
4) The platelets release ASA after activation with thrombin, and this release is absent if pretreatment with dipyridamole is performed. On incubating the platelets with radiolabelled ASA we were able to verify that if this platelet preparation was activated with thrombin, it was possibile to assay the radiolabelled substance in the external medium; this effect is due to the fact that, after activation, the platelets release the contents of the granules to the outside of the cells, such as ADP, ATP, Ca2+, etc. The demonstration that ASA enters the granules is provided by the fact that, in the case of pretreatment of the platelets with dipyridamole, the release of radiolabelled ASA was absent, whereas the release of the other granular components was entirely normal (1.8 ± 0.3 nmoles of ATP released by the platelets pretreated with dipyridamole as against 1.9 ± 0.1 nmoles of ATP released by the control platelets) (see Figure 3);
5) in platelets from megakarocytes in culture in which expression of the channel was suppressed by blocking RNA transcription by means of specific siRNA for MRP4, the platelets treated with ASA presented intracellular ASA and salicylate values which were 159% and 144% higher, respectively, than those of a platelet population that was similar but treated with the vehicle alone (lipofectamine) and incubated with the same concentration and for the same time with ASA.

Another important conclusion which the inventors arrived at through the experiments conducted is that by reducing MRP4-mediated transport it proves possible to enhance the action of ASA in inhibiting COX-1. In fact, when dipyridamole or MK-571 is added prior to treatment with ASA, the production of thromboxane, a prostaglandin produced by platelets as a result of activation of COX-1, induced by thrombin, is less than that of platelets which are aspirinated but not pretreated with MRP4-mediated transport inhibitors. The thrombin-induced production of thromboxane of the aspirinated platelets was 343.7 ng/10⁸ cells, whereas, if the MRP4-mediated transport was inhibited by means of dipyridamole or MK-571, thromboxane production was down to 142.7 ng/10⁸ cells, a 58% reduction, and to 140.22 ng/10⁸ cells, a 59.2% reduction, respectively.

The finding that platelets from megakaryocytes in culture, in which expression of the channel had been suppressed by treatment with siRNA, if aspirinated, presented a 51% reduced production of thromboxane as compared to platelets from megakaryocytes in culture treated with the siRNA vehicle alone clearly demonstrates that, if the platelets do not present this channel, aspirin inhibts COX-1 more effectively.

The correlation between ASA resistance and MRP4 is also evident from the fact that patients who appear to be more resistant to the action of ASA, such as, for example, those undergoing bypass surgery, diabetics and patients on chronic treatment with ASA, have a different platelet MRP4 expression as compared to healthy volunteers (Fig. 4). In fact, in all the tested patients a band related to MRP4 is observed (Jedlitschky et al. Blood 2004, 104, 3603-3610) which is densitometrically higher when compared with healthy controls.

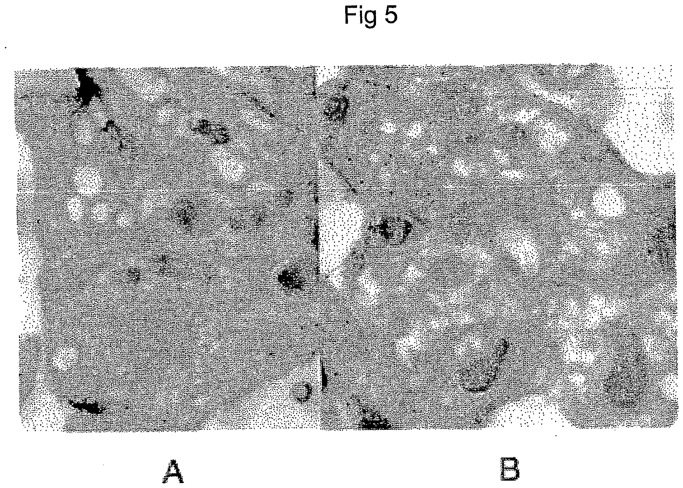

We can hypothesise that in the platelets of resistant patients there is a greater expression of the channel. This hypothesis is confirmed by the experiments conducted using the immunogold technique (Fig. 5A and 5B), where we saw that resistant patients present an increased platelet concentration of the channel with respect to healthy volunteers. As additional confirmation of the fact that the patients that are less sensitive to the action of aspirine in reducing the platelet response, we have verified for them that the platelet MRP4 genic expression, calculated by quantifying the specific mRNA, is increased by 214% in plateles from patients subjected to bypass as compared with platelets from healthy volunteers.

Finally we have demonstrated that by reducing the MRP4 mediated transport in platelets obtained from highly ASA resistant patients, an increase in the therapeutic efficacy of aspirine is obtained.

In fact, in platelets obtained from patients subjected to aortho coronaric bypass and pretreated *in-vitro* with dipirydamole before aspirine, the thromboxane production induced by thrombin was 35% with respect to the platelets treated with aspirine alone (499,8 ± 247,7 ng/10⁸ cells versus 1437,0 ± 549,1 ng/10⁸ cells).

To evaluate the MRP4-mRNA platelet values capable of indicating the patients resistant to aspirin, dosages in healthy volunteers and patients were performed at 5 days from bypass operation and we verified that the healthy volunteers showed values of 21,1±12,8 EM 3,2 mean 23,2 min 4,1 Mx 37,5 UA of theshold cycles of MRP4/GAPDH. It can be hypotesise that values higher than 20 UA, preferably higher than 25 UA, more preferably higher than 30 UA, much more preferably higher than 35 UA are values that identify resistant patients. Considering the pre-analytic and analytic variabilities of the lab data obtained with the kit for mRNA, it is recommended to create your own normal values, that can be obtained after analyzing at least 15 healthy volunteers. As for the kit with western blot, our data demonstrated that resistant patients showed a protein expression higher than 20 % (densitometry carried out with the computer program ImageJ, NIH, Bethesda, USA) in comparison with the controls of the healthy volunteers and patients studied at the same time.

We can hypothesise that the higher expression of these channels reduces the pharmacological effects of aspirine in platelets in that it reduces its intacellular entrapment.

It is for this reason that the identification of patients whose platelets show high levels of MRP4 is of fundamental importance in reducing the level of therapeutic failures linked to aspirin chronic treatment.

The final conclusion is that ASA can be eliminated from platelets by means of MRP4-mediated transport: greater expression of these channels would therefore be responsible for the reduced cellular accumulation of the drug with a consequent reduction of the pharmacological effect. An illustrative but by no means exhaustive list of MRP4s, includes, in addition to dipyridamole [2,6-bis-(diethanolamino)-4,8-dipiperidino-(5,4-d)-pyrimidine] and mopidamole [2,6-bis-(diethanolamino)-8-piperidino-(5,4-d)-pyrimidine]: MK-571; pyrimido-pyrimidine derivatives such as those described in US Patent 4694024; purine analogues suc as the 6-oxo-purines and pyrazol[3,4-d] pirimidines (described in J. Comb. Chem 2007, 9, 210-218); quinoline derivatives, such as mefloquine, amodiaquine, chloroquine, primaquine, quinidine and quinine, cilostazol and quinolinone derivatives, polyphenols, particularly those of a natural origin and those of a synthetic one, such as those described in FEBS J. September 2005, 272 (18): 4725-4740; diclofenac, celecoxib, dl-buthionine-(S,R)-sulphoximine (BSO), probenecide, S-(2,4-dinitrophenyl)-glutathione (DNP-SG), *N*-acetyl-(2,4-dinitrophenyl)cysteine (NAc-DNP-Cys), α-naphthyl-*β*-D-gluco-ronide, *p*-nitrophenyl-*β*-D-glucoronide, flurbiprofen, ibuprofen, indomethacin, indoprofen, ketoprofen, diclofenac, celecoxib, rofecoxib, disulphiram; corresponding derivatives and physiologically acceptable salts of said compounds, and mixtures thereof.

They all exert the action of reducing MRP4-mediated transport, thus enhancing the inhibitory effect of ASA on platelet COX-1.

The concentration of dipyridamole necessary in *in-vitro* studies is high, but could be lower *in vivo* and range from 75 to 500 mg, preferably 75 to 200 mg.

This new mechanism of action of dipyridamole or other inhibitors of MRP4-mediated transport may offer various advantages, such as reducing the effect referred to as ASA resistance, thus enhancing the therapeutic efficacy of this drug in the diseases described above; it may also offer the major advantage of being able to reduce the pharmacologically active doses in all diseases requiring chronic treatment, thus reducing the complications of ASA treatment, and above all its gastrolesivity.

The new-generation molecules which will be capable of reducing MRP4-mediated transport, in addition to reducing the phenomenon of resistance to chemotherapy agents and antiviral agents in oncological diseases, will also offer the advantage, as demonstrated by the inventors, of improving chronic aspirin treatment in all diseases requiring such treatment.

The advantages deriving from the combined use of treatment with ASA plus inhibitors of MRP4-mediated transport are the following:
1) reduction of complications in cardio- and cerebrovascular diseases, obliterating arteriopathies of the peripheral vessels, retinopathy, atrial fibrillation, deep vein thrombosis, cardiac rhythm diseases, thrombocytosis, gestosis of pregnancy, recurrent miscarriages, and ASA treatment of patients with a family history of various types of tumours, such as colon cancer, to reduce the incidence of these diseases;
2) Reduction of cardio- and cerebrovascular accidents (therapeutic failure) in patients on treatment with ASA alone in primary prevention (patients at high risk of atherothrombotic diseases); in secondary prevention (patients previously suffering a thrombotic event);
3) reduction of the daily dose of ASA with a reduction of side effects;
4) possibility of administering ASA to patients who cannot take large amounts of it, due to gastric sensitivity to the drug, but who need treatment for lengthy periods, such as patients affected by chronic inflammatory diseases;
5) possibility of administering ASA at a reduced dose for short periods and thus of reducing the risk of NSAID-induced gastric damage.

The following examples are given to illustrate the invention and must not be considered as limitative of its scope.

### Selection of the patients

The following patients were enroled for the study:
- healthy volunteers who had declared they had not taken any drug in the 15 days before the blood tests;
- patients under chronic aspirin therapy (100-160 mg/ day for at least two months.
- patients who suffered from diabetes mellitus type II, that had been diagnosed according to the International Society of Diabetes guidelines (Diagnosis and classification of diabetes mellitus. Diabetes Care 2006;29 Suppl 1:543-8);
- patients at 5 days from an aortic-coronary- bypass.

### Exclusion criteria

The following patients were excluded from the research:
1) those who had taken, in the last 15 days, drugs that interfered with the platelet function (steroidal and non-steroidal anti-inflammatory drugs, thienopiridines, anticoagulant agents);
2) those who suffered from pathologies that could alter the platelet functionality;
3) those who showed a number of leucocytes in the PRP higher than 0,2x10³ cells/microl.

### Preparation of the biological samples: platelet rich plasma (PRP), platelet poor plasma (PPP)

Venous blood of patients was taken in a test tube with 3,8% sodium citrate (dilution 1:10)

The blood sample with citrate sodium was centrifugated at 200g for 15 minutes at room temperature, thus obtaining as supernatant platelet rich plasma (PRP). The PRP was then centrifugated at 200 g for 5 minutes in order to impove the purification from whole blood in the supernatant.

The PRP was then subjected to platelet count through blood cell count Celltac Auto Nihon Kohden (MEK 8118K). The platelets were then moved away from plasma through centrifugation of PRP, acidified at pH 6,5 with ACD (citric acid, sodium citrate and glucose 1/10 v/v), at 800 x g for 10 min. and resuspended in Tyrode's buffer (CaCl₂ 0.20 g/L, KC1 0.20 g/L, MgCl₂ g/L, MgCl₂ g/L, NaCl 8.00 g/L, NaH₂PO₄*H₂O), at pH 7.35 and charged with glucose and albumin. The preparation of a dense rich platelet granule suspension was made according to the method reported by (Fukami MH et al. J. Cell. Biol. 1978, 77, 389-99) and the degree of purity was verified through mepacrine Fluorimetric probe and the purity percentage was checked through the cytofluorimeter. If the concentration of the dense granules compared to the other organelles was lower than 80%, the suspension of platelet granules was not used for the study.

### Treatment of platelets with aspirin

The resuspended platelets in the Tyrode's buffer at the concentration of 2.5x10⁸ cells/ml were treated with aspirin (50 microM) at 10°C for 15 min and then kept at 37°C or at 4°C for 15 min.

### Reduction of the MRP4 mediated transport

MRP4 inhibitors were used such as Dipyridamole and MK571 (*(E)*-3-[[[3-[2-(7-chloro-2-quinolinyl)ethenyl]phenyl]-[[3-dimethylamino)-3-oxo-propyl]-thio]-methyl]thio]-propanoic acid), both at 100 microM and incubated 30 min at RT before aspirin addition.

In the rich platelet dense granule suspension, ATP and ASA were added to the preparation, then it was kept at 37°C for 15 min so a to reduce the transpost mediated by MRP4; the suspension was pretreated either with apyrase, an ATP scavenger substance or with Dipyridamole 30 min before adding aspirin, or the treatment with aspirin was made at 4°C.

### Measurement of the cytosolic concentrations of ASA and of its metabolite, salicylic acid (SA)

The entrapment of aspirin inside platelets or in the dense granules was measured through the method of Gaspari and Locatelli (Ther Drug Monit. 1987, 9, 243-7).

### Platelet secretion of ASA marked with (¹⁴C), in thrombin activated platelets

Aspirin labelled with ¹⁴C (American Radiolabeled Chemicals, Inc. Saint Louis, USA) was added to the platelet suspension for 15 min at 37°C. After removing the exces of the substance marked by double centrifugation, the platelets were activated with Thrombin (0,2 U/ml) and then centrifugated. The reduction of the transport mediated by MRP4 was made through the preincubation of the platelet preparation with Dipyridamole 30 min before aspirin addition. The results are reported by evaluating the percentage of the radioactive present in the supernatant compared to the total radioactivity of the platelet preparation. ATP secretion was analyzed as a control through the luminometric method with commercial kit (Chronolog, Hevertown, PA, USA).

### Evaluation of the biologic efficacy of aspirin

The evaluation of the biologic efficacy of aspirin in platelets was made by studying Thromboxane A2 production, a specific marker of the cyclooxygenase pathway in platelets. Aspirin and/or Dipyridamole treated platelets (see above) were activated with thrombin 1U/ml for 1 h at 37°C, and then centrifugated 1 min at 8.000 g. Thromboxane B2, a stable metabolite of TXA₂, was measured in the supernatant through a commercial ELISA kit (Cayman, chemicals Co).

### Evaluation of the platelet expression of MRP4 through Western Blot

The platelet expression of the MRP4 was studied through an electrophoretic technique and the subsequent transfer of protein on membrane through Western Blotting, from a volume of platelet rich plasma (PRP) containing 5x10⁸ platelets.

After carefully removing the supernatant, two platelet washings were made through suspension in Tyrode's buffer (pH 6.35) and centrifugation at 8000 g for 2 min.

The platelet pellet was resuspended in 200 µl lysis buffer (RIPA buffer) and kept in ice for one hour.

Then, the Laemly buffer solution 5x and beta-mercaptoethanol 5% were added and the sample was kept at 100°C for 5 min. After 15 min in ice, the sample was centrifugated at 12000 g for 4 min and the supernatant was then used for the electrophoresis on a polyacrilamide gel at 4-12% containing SDS 1% (SDS-PAGE). A marker containing a mixture of coloured proteins at known molecolar weight were used in order to identify the proteins dimensions.

Then the proteins were transferred through Western Blot on a cellulose membrane.

The identification of the protein was made through primary polyclonal antibodies heading against the MRP4 (Santa-Criz and Alexis), and secondary antibodies conjugated with the peroxidase and then treated with ECL (a solution containing a chemoluminescent substrate), for the production of light that can be detected with photographic film.

### Evaluation of the MRP4 platelet expression through Immunogold micrography

Platelets were fixed with paraformaldehyde (2%) and embedded in 10% gelatin and solidified on ice. After the preparation was frozen in liquid nitrogen and cryosectioned using an Ultracut EM FC (Leica). Ultrathin cryosections were thus treated with polyclonal-antibody anti MRP4 (Alexis) followed by the addition of 10 nm diameter proteinA- colloidal gold conjugates of. After labeling all ultrathin cryosections, the preparations were fixed in 1% glutharaldheyd and stained with a solution of methyl cellulose (2%) and uranyl acetate (0,4%) to observe it under electron-microscopy.

### Platelet preparation from megakariocytes in culture treated with si-MRNA,

Platelets from megakariocytes in culture were prepared according to the method by Guerriero et al (Journal of Cell Science 119, 744-752, 2006).The transfection with siRNA was made according to the method by Dharmacon Research using the SMARTpool product of four siRNAs for MRP4. 3 days after transfection the produced platelets from megakariocytes were isolated through centrifugation, then aspirinated, and then aspirin entrapment and the pharmachological efficacy were measured (see above). Platelets from megakariocytes treated with only vehicle used for the transfection of siRNA were utilized as a control.

### Measurement of the platelet MRP4-mRNA. concentration

Platelet DNA prepared with the reversal-PCR method according to the method by Paul el al. (Methods Mol Biol. 2004;273:435-54) was amplified by using primers anti MRP4 e GAPDH (Glyceraldehyde-3-phosphate dehydrogenase), both obtained from Applied Biosystems, at standard cyclic thermic conditions. In parallel standard cDNA curves have been made for the quantization of the results. The results are reported as expression of the levels of MRP4-mRNA standardized with GAPDH-mRNA. Other mRNA constant platelet standards can also be used.

## Claims

1. A method to determine in-vitro ASA (acetylsalicylic acid) resistance in subjects, based on determining the levels of MRP4 (Multidrug Resistance Protein 4) in platelets, such method comprising the following steps:
(i) purify a sample of a biological fluid to obtain a platelet sample;
(ii) either
(a) put it into contact with an anti MRP4 mono- or poly-clonal antibody for a time sufficient for the formation of a complex between antigen MRP4 and said antibody, and detect and measure such complex by Western Blot; or
(b) measure platelet MRP4-mRNA concentration by Southern Blot or Real Time PCR;
said subjects being ASA resistant if presenting a higher platelet expression of MRP4 compared to healthy controls.

2. The method according to claim 1, wherein said biological fluid is peripheral venous blood.

3. The method according to claims 1 or 2, wherein ASA resistance is due to chronic treatment with ASA or to genetic causes.

4. Use of a kit for performing the method according to claims 1 to 3, the kit comprising, in addition to the material to perform the test, also anti-MRP4 antibodies for the evaluation test through Western Blot; or restriction enzymes specific for MRP4 to be quantitatively analyzed, in order to determine the platelet mRNA through Southern Blot or RT-PCR.

5. Use according to claim 4, further comprising a standard preparation of MRP4.

6. Use of a composition comprising ASA, its derivatives and physiologically acceptable salts, in combination with MRP4 channel inhibitors for the preparation of a medicament for the treatment of patients in need of ASA therapy and diagnosed as being ASA resistant and showing higher platelet expression of MRP4 compared to healthy controls, comprising determining whether a patient is ASA resistant by performing the method of any of claims 1-3, wherein the MRP4 channel inhibitor is selected from the group consisting of dipyridamole; mopidamole; MK-571; mefloquine, amocliaquine, chloroquine, primaquine, quinidine and quinine; dl-buthionine-(S,R)-sulphoximine; probenecide; S-(2,4-dinitrophenyl)glutathione; N-acetyl-(2,4-dinitro-phenyl) cysteine; α-naphthyl-β-D-glucoronide; p-nitrophenyl-β-D-glucoronide; flurbiprofen; ibuprofen; indomethacin;
indoprofen; ketoprofen; diclofenac; disulphiram; corresponding physiologically acceptable salts of said compounds, and mixtures thereof.

7. Use according to claim 6, wherein ASA and the inhibitor are prepared in formulations packaged as separate administration forms of the active ingredients, for the co-ordinate simultaneous or successive administration of the active ingredients.

8. Use according to claim 6 or 7, wherein the composition of ASA and inhibitors additionally comprises excipients, diluents, vehicles and adjuvants.

9. Use according to claim 8, wherein the excipients, diluents, vehicles and adjuvants are selected from the group consisting of water, saline solution, glycerol, ethanol, emulsifying agents, buffer substances and preservatives.

10. Use according to claims 6-9, wherein the composition is prepared in solid, semisolid or liquid form, in the form of lozenges, capsules, pills, tablets, pastilles, granulates, syrups, ampoules, drops, solutions, suspensions, emulsions, in unitary or separate dosage forms, ready-to-use syrups or ex tempore units, creams and gels.

11. Use according to claims 6-10, wherein the composition is prepared in formulations that can be administered orally, parenterally, rectally, by topical administration or by intramuscular, subcutaneous, transdermal or intravenous administration.

12. Use according to claims 6-11, wherein the combination of ASA and the MRP4 inhibitor dipyridamole is administered in amounts ranging, respectively, from 75 to 150 mg/day in the case of ASA and from 75 to 500 mg/day, or 75 to 200 mg/day, in the case of dipyridamole.

13. Use according to claims 6-11, wherein the combination of ASA plus the MRP4 inhibitor dipyridamole is administered in daily doses of 40 mg of ASA and 75 mg of dipyridamole.

## Patentansprüche

1. Verfahren zur Bestimmung der *in-vitro*-ASS-(Acetylsalicylsäure)-Resistenz bei Patienten, basierend auf der Bestimmung der MRP4-(Multidrug Resistance Protein 4)-Spiegel in Blutplättchen, wobei das Verfahren die folgenden Schritte umfasst:
(i) Aufreinigen einer Probe einer biologischen Flüssigkeit, um eine Blutplättchenprobe zu erhalten;
(iii) entweder
(a) In-Kontakt-Bringen der Probe mit einem monoklonalen oder polyklonalen Anti-MRP4-Antikörper für eine Zeitspanne, die ausreichend zur Bildung eines Komplexes zwischen dem Antigen MRP4 und dem Antikörper ist, und Nachweisen und Messen eines solchen Komplexes mittels Western Blot; oder
(b) Messen der Konzentration der MRP4-mRNA der Blutplättchen mittels Southern Blot oder Echtzeit-PCR;
wobei die Patienten ASS-resistent sind, wenn sie eine höhere MRP4-Expression in Blutplättchen aufweisen verglichen mit gesunden Kontrollen.

2. Verfahren gemäß Anspruch 1, wobei die biologische Flüssigkeit venöses peripheres Blut ist.

3. Verfahren gemäß Anspruch 1 oder 2, wobei die ASS-Resistenz auf einer chronischen Behandlung mit ASS beruht oder genetische Ursachen hat.

4. Verwendung eines Kits für die Durchführung des Verfahrens gemäß den Ansprüchen 1 bis 3, wobei das Kit zusätzlich zu dem Material, um den Test durchzuführen, auch Anti-MRP4-Antikörper zur Auswertung des Tests mittels Western Blot umfasst; oder Restriktionsenzyme, die spezifisch sind für das quantititiv zu analysierende MRP4, um die Blutplättchen-mRNA mittels Southern Blot oder RT-PCR zu bestimmen.

5. Verwendung gemäß Anspruch 4, weiterhin umfassend ein Standardpräparat von MRP4.

6. Verwendung einer Zusammensetzung enthaltend ASS, deren Derivate und physiologisch verträglichen Salze, in Kombination mit MRP4-Kanal-Hemmern zur Herstellung eines Arzneimittels zur Behandlung von Patienten, die der ASS-Behandlung bedürfen und als ASS-resistent diagnostiziert wurden und eine höhere Blutplättchen-Expression von MRP4 zeigen verglichen mit gesunden Kontrollen, umfassend die Bestimmung, ob ein Patient ASS-resistent ist, mittels Durchführung des Verfahrens gemäß irgendeinem der Ansprüche 1 bis 3, wobei der MRP4-Kanal-Hemmer ausgewählt ist aus der Gruppe bestehend aus Dipyridamol; Mopidamol; MK-571; Mefloquin, Amodiaquin, Chloroquin, Primaquin, Chinidin und Chinin; dl-Buthionin-(S,R)-sulphoximin; Probenecid; S-(2,4-Dinitrophenyl)-Glutathion; N-Acetyl-(2,4-dinitrophenyl)-Cystein; α-Naphthyl-β-D-Glucoronid; p-Nitrophenyl-β-D-Glucoronid; Flurbiprofen; Ibuprofen; Indometacin; Indoprofen; Ketoprofen; Diclofenac; Disulfiram; entsprechenden physiologisch verträglichen Salzen der genannten Verbindungen, und Mischungen davon.

7. Verwendung gemäß Anspruch 6, wobei ASS und der Hemmer in Formulierungen hergestellt werden, die als getrennte Darreichungsformen der Wirkstoffe verpackt werden, für die koordinierte, gleichzeitige oder nacheinander folgende Verabreichung der Wirkstoffe.

8. Verwendung gemäß Anspruch 6 oder 7, wobei die Zusammensetzung von ASS und Hemmern zusätzlich Hilfsstoffe, Verdünnungsmittel, Trägerstoffe und Adjuvantien umfasst.

9. Verwendung gemäß Anspruch 8, wobei die Hilfsstoffe, Verdünnungsmittel, Trägerstoffe und Adjuvantien ausgewählt sind aus der Gruppe bestehend aus Wasser, Salzlösung, Glycerin, Ethanol, Emulgatoren, Puffersubstanzen und Konservierungsmitteln.

10. Verwendung gemäß den Ansprüchen 6 bis 9, wobei die Zusammensetzung in fester, halbfester oder flüssiger Form hergestellt wird, in Form von Lutschtabletten, Kapseln, Pillen, Tabletten, Pastillen, Granulaten, Sirupen, Ampullen, Tropfen, Lösungen, Suspensionen, Emulsionen, in einheitlichen oder getrennten Darreichungsformen, gebrauchsfertigen Sirupen oder Ex-tempore-Einheiten, Cremes und Gelen.

11. Verwendung gemäß den Ansprüchen 6 bis 10, wobei die Zusammensetzung in Formulierungen zubereitet wird, die oral, parenteral, rektal, durch topische Verabreichung oder durch intramuskuläre, subkutane, transdermale oder intravenöse Verabreichung verabreicht werden können.

12. Verwendung gemäß den Ansprüchen 6 bis 11, wobei die Kombination von ASS und dem MRP4-Hemmer Dipyridamol in Mengen verabreicht wird im Bereich jeweils 75 bis 150 mg/Tag im Fall von ASS und 75 bis 500 mg/Tag, oder 75 bis 200 mg/Tag, im Falle von Dipyridamol.

13. Verwendung gemäß den Ansprüchen 6 bis 11, wobei die Kombination von ASS plus dem MRP4-Hemmer Dipyridamol in täglichen Dosen von 40 mg ASS und 75 mg Dipyridamol verabreicht wird.

## Revendications

1. Méthode pour déterminer la résistance à l'ASA (acide acétylsalicylique) *in vitro* chez des sujets, sur la base de la détermination des niveaux de MRP4 (protéine 4 de multirésistance aux médicaments) dans des plaquettes, une telle méthode comprenant les étapes suivantes :
(i) purifier un échantillon d'un fluide biologique pour obtenir un échantillon de plaquette ;
(ii) soit
(a) le mettre en contact avec un anticorps mono-ou poly-clonal anti-MPR4 pendant un temps suffisant pour la formation d'un complexe entre un antigène MRP4 et ledit anticorps, et détecter et mesurer un tel complexe par buvardage de Western ; soit
(b) mesurer la concentration en MRP4-ARNm des plaquettes par buvardage de Southern ou PCR en temps réel ;
lesdits sujets étant résistants à l'ASA s'ils présentent une expression de plaquettes supérieure de MRP4 en comparaison à des témoins sains.

2. Méthode selon la revendication 1, dans laquelle ledit fluide biologique est du sang veineux périphérique.

3. Méthode selon les revendications 1 ou 2, dans laquelle la résistance à l'ASA est due à un traitement chronique avec l'ASA ou à des causes génétiques.

4. Utilisation d'un kit pour réaliser la méthode selon les revendications 1 à 3, le kit comprenant également, en plus du matériel pour réaliser l'essai, des anticorps anti-MRP4 pour l'essai d'évaluation au moyen d'un buvardage de Western ; ou des enzymes de restriction spécifiques à la MRP4 à analyser quantitativement, afin de déterminer l'ARNm de plaquettes au moyen d'un buvardage de Southern ou d'une RT-PCR.

5. Utilisation selon la revendication 4, comprenant en outre une préparation standard de MRP4.

6. Utilisation d'une composition comprenant de l'ASA, ses dérivés ou des sels physiologiquement acceptables, en combinaison avec des inhibiteurs de canal de MRP4 pour la préparation d'un médicament pour le traitement de patients nécessitant une thérapie à l'ASA et ayant été diagnostiqués comme étant résistants à l'ASA et présentant une expression de plaquettes supérieure de MRP4 en comparaison à des témoins sains, comprenant le fait de déterminer si un patient est résistant à l'ASA en réalisant la méthode de l'une quelconque des revendications 1 à 3, dans laquelle l'inhibiteur de canal de MRP4 est choisi dans le groupe consistant en le dipyridamole ; le mopidamole ; MK-571 ; la méfloquine, l'amocliaquine, la chloroquine, la primaquine, la quinidine et la quinine ; la dl-buthionine-(S,R)-sulfoximine ; le probénécide ; le S-(2,4-dinitrophényl)glutathion ; la N-acétyl-(2,4-dinitro-phényl)cystéine ; l'α-naphtyl-β-D-glucoronide ; le p-nitrophényl-β-D-glucoronide ; le flurbiprofène ; l'ibuprofène ; l'indométacine ; l'indoprofène ; le kétoprofène ; le diclofénac ; le disulfirame ; des sels physiologiquement acceptables correspondants desdits composés, et leurs mélanges.

7. Utilisation selon la revendication 6, dans laquelle l'ASA et l'inhibiteur sont préparés dans des formulations emballées comme des formes d'administration séparées des ingrédients actifs, pour l'administration coordonnée simultanée ou successive des ingrédients actifs.

8. Utilisation selon la revendication 6 ou 7, dans laquelle la composition de l'ASA et des inhibiteurs comprend en outre des excipients, des diluants, des véhicules et des adjuvants.

9. Utilisation selon la revendication 8, dans laquelle les excipients, les diluants, les véhicules et les adjuvants sont choisis dans le groupe consistant en l'eau, une solution saline, le glycérol, l'éthanol, des agents émulsifiants, des substances tampons et des conservateurs.

10. Utilisation selon les revendications 6 à 9, dans laquelle la composition est préparée sous forme solide, semi-solide ou liquide, sous la forme de tablettes, capsules, pilules, comprimés, pastilles, granulés, sirops, ampoules, gouttes, solutions, suspensions, émulsions, dans des formes posologiques unitaires ou séparées, de sirops prêts à l'emploi ou d'unités, de crèmes et de gels extemporanés.

11. Utilisation selon les revendications 6 à 10, dans laquelle la composition est préparée dans des formulations qui peuvent être administrées par voie orale, parentérale, rectale, par administration topique ou par administration intramusculaire, sous-cutanée, transdermique ou intraveineuse.

12. Utilisation selon les revendications 6 à 11, dans laquelle la combinaison de l'ASA et de l'inhibiteur de MRP4 dipyridamole est administrée dans des quantités allant, respectivement, de 75 à 150 mg/jour dans le cas de l'ASA et de 75 à 500 mg/jour, ou 75 à 200 mg/jour, dans le cas du dipyridamole.

13. Utilisation selon les revendications 6 à 11, dans laquelle la combinaison de l'ASA plus l'inhibiteur de MRP4 dipyridamole est administrée dans des doses quotidiennes de 40 mg d'ASA et de 75 mg de dipyridamole.
